# FASCICULE DE BREVET EUROPEEN

(11) **EP 1 404 211 B1**
(45) Date de publication et mention de la délivrance du brevet: **10.08.2011**
(21) Numéro de dépôt: 03735779.5
(22) Date de dépôt: 14.03.2003
(51) Int. Cl.: A61B 1/267

(54) **LARYNGOSCOPE AVEC UNE LAME ET UN MANCHE**
LARYNGOSKOP MIT EINER KLINGE UND EINEM HANDGRIFF
LARYNGOSCOPE WITH BLADE AND HANDLE

(30) Priorité: 15.03.2002 FR 0203226
(43) Date de publication de la demande: 07.04.2004
(73) Titulaire: Vygon, F-95440 Ecouen (FR)
(72) Inventeur: DALLE, Valéry, F-60270 Gouvieux (FR); EUDES, Yvan, F-95460 Ezanville (FR); TRELLU, Eric, F-60260 Lamorlaye (FR)
(74) Mandataire: Texier, Christian
(86) Numéro de dépôt international: PCT/FR2003/000823
(87) Numéro de publication internationale: WO 2003/077737

(56) Documents cités:
- EP-A- 0 169 497
- EP-A- 0 653 180
- WO-A-97/30626
- GB-A- 2 191 949
- US-A- 5 651 760

## Description

L'invention concerne les laryngoscopes du type comprenant une lame non métallique à usage unique ou peu répété et un manche à usage intensif, la lame et le manche étant munis de moyens respectifs coopérant pour permettre de monter de façon détachable la lame sur le manche dans au moins une position prédéfinie.

Les moyens de montage de la lame sur le manche comprennent des moyens d'enclenchement qui permettent de mettre la lame en prise sur le manche et des moyens de blocage à effet ressort qui agissent pour maintenir la, lame dans la position choisie mais qui peuvent être rendus inactifs par une manipulation appropriée de la lame pour pouvoir séparer la lame du manche après usage.

La publication EP 0 169 497 décrit une réalisation; dans laquelle :
- les moyens d'enclenchement comportent sur le manche une cavité située à une extrémité du manche, ouverte vers l'extérieur et contenant un barreau placé en travers de la cavité, et comportent sur la lame un crochet formé sur un talon à une extrémité de la lame pour venir en prise pivotante sur le barreau lorsque ce talon est introduit dans la cavité du manche.
- les moyens de blocage sont constitués par une paroi prévue sur le manche en bordure de la cavité qui reçoit le talon de la lame pour venir en contact de coincement avec une paroi en vis-à-vis de ce talon sous la poussée d'une lame de ressort fixée au manche.

S'agissant d'un manche à usage répété, le risque existe d'une fatigue de la lame du ressort qui deviendrait moins performante pour assurer le blocage de la lame.

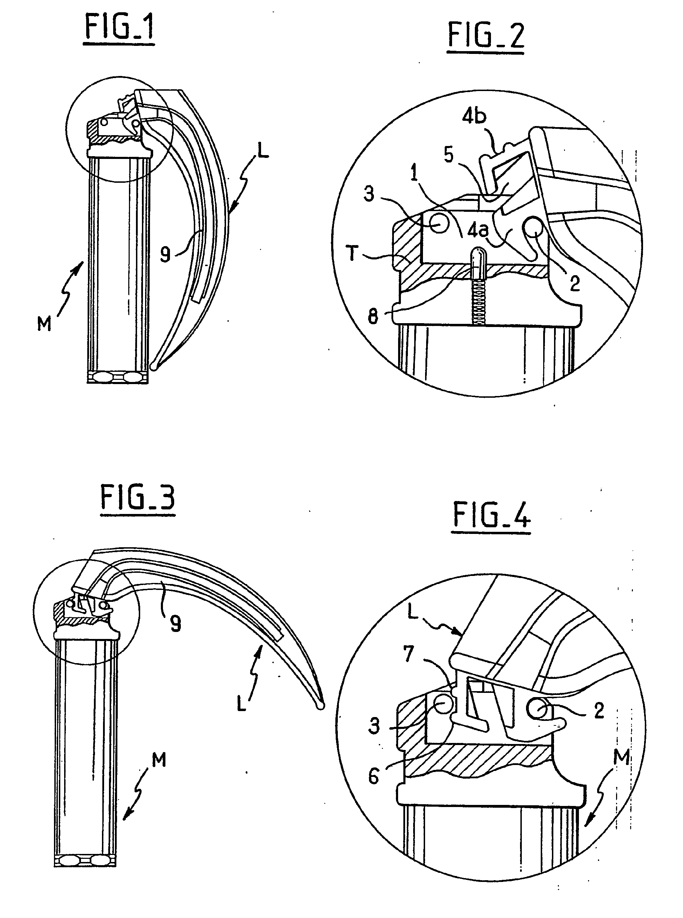

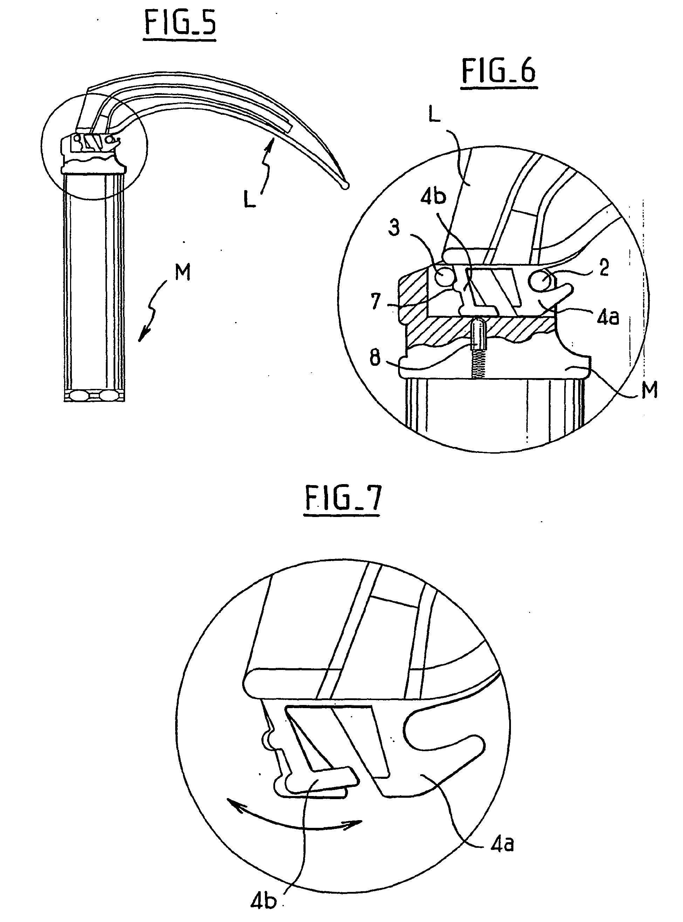

La publication GB 2 191 949 décrit des laryngoscopes qui comprennent un adaptateur entre la lame et le manche. Dans une réalisation (figs. 4 et 5) où les moyens d'enclenchement du manche sur l'adaptateur comprennent un barreau et où les moyens d'enclenchement de la lame sur l'adaptateur comprennent un crochet, les moyens de blocage du manche sur l'adaptateur et les moyens de blocage de la lame sur l'adaptateur comprennent chaque fois une bille poussée par un ressort. Dans une autre réalisation, où les moyens d'enclenchement de la lame sur l'adaptateur comprennent une vis qui traverse un talon de la lame et l'adaptateur, les moyens de blocage de la lame sont constitués par une lame de ressort fixée sur le talon de la lame et qui vient en butée dans une cavité latérale de l'adaptateur. Des moyens d'enclenchement et des moyens de blocage similaires sont prévus sur le manche.

La présente invention a pour but de fournir un laryngoscope de conception beaucoup moins coûteuse, compatible avec l'emploi d'une lame à usage unique.

L'invention vise à assurer le blocage de la lame sans compromettre le coût de fabrication de la lame et! sans qu'un usage répété du manche puisse compromettre l'efficacité du blocage.

L'invention est un laryngoscope tel que défini dans la revendication 1.

On y parvient essentiellement, selon l'invention, en formant sur le talon de la lame une paroi élastiquement flexible située en vis-à-vis et à distance du crochet du talon en sorte que cette paroi soit contrainte à s'incliner légèrement à force vers le crochet lorsque le talon est introduit dans la cavité du manche et que la paroi vient au contact d'une butée fixe du manche, cette inclinaison engendrant une force de rappel visant à redresser la paroi, ce qui assure une pression de contact de la paroi avec ladite partie du manche.

Ainsi, selon l'invention, l'effet ressort peut être dû à un simple crevé du talon, lequel peut venir de moulage ou d'un usinage simple après moulage.

Cette conception permet aussi de simplifier le manche de la lame en prévoyant comme butée de contact avec la paroi flexible du talon, un simple barreau fixe parallèle au barreau entouré par le crochet.

Ainsi, les moyens de verrouillage comprennent sur le manche un moyen fixe peu susceptible d'usure malgré une utilisation répétée du manche et, sur la lame, un moyen flexible qui, même s'il était susceptible d'usure à la longue, échappe à ce handicap puisque la lame est à usage unique ou peu répété.

On décrira ci-après un exemple de réalisation d'un laryngoscope comportant une lame et un manche conformes à la présente invention, en référence aux figures du dessin joint sur lequel :
- La figure 1 est une vue en perspective de la lame sur le manche alors que le crochet du talon de la lame vient d'être mis en prise sur un barreau du manche ;
- La figure 2 est une vue agrandie de la partie de la figure 1, délimitée par un cercle ;
- Les figures 3 et 4 sont similaires respectivement à la figure 1 et à la figure 2, alors que la lame a pivote sur ledit barreau jusque dans une première position de verrouillage ;
- Les figures 5 et 6 sont similaires respectivement à la figure 1 et à la figure 2, alors que la lame a pivoté sur ledit barreau jusque dans une deuxième position de verrouillage, et
- La figure 7 est une vue en détail agrandie de la paroi flexible du talon de la lame.

On a représenté sur les figures la lame L et le manche M d'un exemple de laryngoscope conforme à l'invention.

Le manche (M) est un cylindre métallique creux préhensible qui présente une extrémité fermée par une tête T vissée sur le cylindre et qui détermine une cavité (1) ouverte vers l'extérieur et dans laquelle se trouvent un premier barreau (2) et un second barreau (3) qui sont rigides et fixes et disposés parallèlement l'un à l'autre en travers de la cavité.

La lame (L), en résine de synthèse et conformée pour pouvoir être introduite dans la bouche afin de plaquer la langue contre la mâchoire inférieure et de soulever l'épiglotte, présente à une extrémité un talon (4) constitué par un crochet (4a) et une paroi nervurée flexible (4b) séparée du crochet par un espace vide (5). La paroi nervurée présente deux nervures parallèles (6,7).

Le talon est dimensionné pour pouvoir pénétrer à force dans la cavité de la tête du manche, avec le crochet en prise sur le barreau (2) et avec la paroi nervurée flexible au contact de l'autre barreau (3), avec les nervures parallèles aux barreaux.

Dans un premier temps (figures 1 et 2) le crochet est en prise avec le premier barreau et la paroi nervurée est encore à l'extérieur du creux de la tête.

Dans un second temps (figures 3 et 4), la première nervure (6) de la paroi nervurée a passé le second barreau (3) et la lame est maintenue dans une première position par la coopération du second barreau avec la paroi nervurée, l'axe se trouvant entre les deux nervures (6,7) de cette paroi.

Dans un troisième temps (figures 4 et 5) la deuxième nervure (7) de la paroi nervurée a passé le second axe (3) et la lame est bloquée dans une deuxième position.

Dans la première position et encore davantage dans la deuxième position, le débattement à force de la paroi flexible du talon de la lame sous l'effet du second barreau crée une force de maintien de la lame en position.

La figure 7 est une vue schématique qui montre le débattement de la paroi flexible.

Le manche contient une batterie électrique pour alimenter une ampoule sous la commande d'un contact électrique en saillie élastique dans ladite cavité et la lame présente un conduit de lumière apte à guider la lumière de ladite ampoule le long de la lame jusqu'à une sortie de lumière lorsque la lame est montée sur le manche et que le talon repousse ledit contact électrique pour provoquer l'éclairage de ladite ampoule.

Le contact électrique a été schématisé en (8) et le conduit en (9) sur les figures. Il n'est pas nécessaire de décrire plus en détails ces moyens qui sont connus en soi et qui ne sont pas l'objet de l'invention.

L'invention n'est pas limitée à cet exemple de réalisation.

## Revendications

1. Laryngoscope comprenant :
■ un manche (M) à usage répété présentant à une extrémité une cavité (1), un barreau fixe transversal (2) dans cette cavité et une butée fixe (3) ; et,
■ une lame (L) non métallique (L) à usage unique comportant un talon (4) pour le montage de la lame dans la cavité (1) formée à l'extrémité du manche de laryngoscope, lequel talon (4) présente une partie (4a) en forme de crochet pour venir en prise avec le barreau (2) présent transversalement dans cette cavité, le talon (4) présentant en vis-à-vis et à distance du crochet une paroi élastiquement flexible (4b) destinée à s'incliner vers la partie (4a) en forme de crochet pour assurer un blocage du talon (4) dans ladite cavité (1) pour le maintien de la lame (L) dans une position définie par rapport au manche (M) lorsque la paroi flexible est contrainte à s'incliner vers la partie (4a) en forme de crochet sous l'effet d'un contact de la paroi avec la butée fixe (3) située sur le manche (M), ladite butée fixe (3) étant un second barreau disposé transversalement dans la cavité (1).

2. Laryngoscope selon la revendication 1 dont ladite paroi flexible présente deux nervures (6,7) qui définissent deux positions de maintien pour la lame.

3. Laryngoscope selon l'une des revendications 1 et 2, dont la lame est fabriquée en résine de synthèse.

4. Laryngoscope selon l'une des revendications 1 à 3, dont le manche (11) est un tube apte à contenir une batterie électrique pour alimenter une ampoule sous la commande d'un contact électrique (8) en saillie élastique dans ladite cavité et dont la lame (L) présente un conduit de lumière (9) apte à guider la lumière de ladite ampoule le long de la lame jusqu'à une sortie de lumière lorsque la lame est montée sur le manche et que le talon de la lame repousse ledit contact électrique pour provoquer l'éclairage de ladite ampoule.

## Claims

1. A laryngoscope comprising:
■ a handle (M) for repeated use having at one end a cavity (1), a transverse fixed bar (2) in this cavity and a fixed abutment (3); and,
■ a non-metal strip (L) for single use including a heel (4) for mounting the strip in the cavity (1) formed at the end of the laryngoscope handle, said heel (4) has a hook-shaped portion (4a) in order to engage with the bar (2) present transversely in this cavity, the heel (4) having, facing and at a distance from the hook, an elastically flexible wall (4b) intended to tilt towards the hook-shaped portion (4a) in order to ensure blocking of the heel (4) in said cavity (1) for maintaining the strip (L) in a defined position relatively to the handle (M) when the flexible wall is forced to tilt towards the hook-shaped portion (4a) under the effect of contact of the wall with the fixed abutment (3) on the handle (M), said fixed abutment (3) being a second bar positioned transversely in the cavity (1).

2. The laryngoscope according to claim 1, said flexible wall of which has two ribs (6, 7) which define two holding positions for the strip.

3. The laryngoscope according to one of claims 1 and 2, the strip of which is made in synthetic resin.

4. The laryngoscope according to one of claims 1 to 3, the handle (11) of which is a tube capable of containing an electric battery for powering a bulb under the control of an electric contact (8) elastically protruding in said cavity and the strip (L) of which has an optical guide (9) capable of guiding light from said bulb along the strip up to an optical output when the strip is mounted on the handle and the heel of the strip pushes back said electric contact in order to cause illumination of said bulb.

## Patentansprüche

1. Laryngoskop mit:
- einem Handgriff (M) zur wiederholten Verwendung mit einem Hohlraum (1) an einem Ende, einem stationären transversalen Stab (2) in diesem Hohlraum und einem stationären Widerlager (3); und
- einer nicht-metallischen Klinge (L) zur einmaligen Verwendung mit einer Ferse (4) zum Einsetzen der Klinge in den an dem Ende des Handgriffs des Laryngoskops gebildeten Hohlraum (1), wobei die Ferse (4) einen hakenförmigen Teil (4a) zum Eingriff mit dem transversal in diesem Hohlraum vorhandenen Stab (2) aufweist, wobei die Ferse (4) gegenüber und in einem Abstand von dem Haken eine elastisch flexible Wand (4b) aufweist, die vorgesehen ist, sich zu dem hakenförmigen Teil (4a) hin zu neigen, um eine Verriegelung der Ferse (4) in dem Hohlraum (1) sicherzustellen, um die Klinge (L) in einer definierten Position bezüglich des Handgriffs (M) zu halten, wenn unter der Wirkung eines Kontakts der Wand mit dem an dem Handgriff (M) angeordneten stationären Widerlager die flexible Wand gezwungen wird, sich zu dem hakenförmigen Teil (4a) zu neigen, wobei das stationäre Widerlager (3) ein zweiter transversal in dem Hohlraum (1) angeordneter Stab ist.

2. Laryngoskop nach Anspruch 1, dessen flexible Wand zwei Rippen (6,7) aufweist, die zwei Haltepositionen für die Klinge definieren.

3. Laryngoskop nach einem der Ansprüche 1 und 2, dessen Klinge aus einem Kunstharz hergestellt ist.

4. Laryngoskop nach einem der Ansprüche 1 bis 3, dessen Handgriff (11) ein Rohr ist, um eine elektrische Batterie aufzunehmen, um unter Steuerung eines in den Hohlraum elastisch vorspringenden elektrischen Kontakts (8) eine Lampe zu versorgen, und dessen Klinge (L) einen Lichtleiter (9) aufweist, um das Licht der Lampe entlang der Klinge bis zu einem Lichtausgang zu leiten, wenn die Klinge an dem Handgriff angebracht ist, wobei die Ferse der Klinge den elektrischen Kontakt zurückdrückt, um das Leuchten der Lampe zu bewirken.
